**Europäisches Patentamt**

**European Patent Office**

**Office européen des brevets**

(11) Veröffentlichungsnummer: **0 653 413 A2**

(12) **EUROPÄISCHE PATENTANMELDUNG**

(21) Anmeldenummer: **95100118.9**

(22) Anmeldetag: **18.08.90**

(51) Int. Cl.6: **C07C 59/125**, C07C 59/13, C07C 59/305, C07C 59/31, C07C 51/235

Diese Anmeldung ist am 05 - 01 - 1995 als Teilanmeldung zu der unter INID-Kode 60 erwähnten Anmeldung eingereicht worden.

(30) Priorität: **26.08.89 DE 3928310**

(43) Veröffentlichungstag der Anmeldung: **17.05.95 Patentblatt 95/20**

(60) Veröffentlichungsnummer der früheren Anmeldung nach Art. 76 EPÜ: **0 489 097**

(84) Benannte Vertragsstaaten: **AT BE CH DE DK FR GB IT LI NL**

(71) Anmelder: **HOECHST AKTIENGESELLSCHAFT Brüningstrasse 50 D-65929 Frankfurt am Main (DE)**

(72) Erfinder: **Schönwälder, Karl-Heinz, Dr. Am Feldbergblick 6 D-65779 Kelkheim (DE)**
Erfinder: **Leupold, Ernst Ingo, Dr. Auf der Erlenwiese 61 D-61267 Neu-Anspach (DE)**
Erfinder: **Gohla, Werner Rathausstrasse 73 D-53859 Niederkassel (DE)**
Erfinder: **Dany, Franz-Josef, Dr. Heddinghovenerstrasse 47 D-50374 Erftstadt (DE)**
Erfinder: **Schlingmann, Merten, Prof. Dr. Schneidhainer Strasse 32a D-61462 Königstein (DE)**

(54) **Waschmittel enthaltende Äthercarbonsäuren.**

(57) Waschmittel enthaltend Äthercarbonsäuren, die durch Oxalkylierung von Kohlenhydraten und deren Derivaten und anschließende Oxidation hergestellt werden, indem man die Kohlenhydrate oder deren Derivate in üblicher Weise entweder unmittelbar äthoxyliert oder zunächst mit einem höheren Alkylenoxid alkoxyliert und dann äthoxyliert und die dabei erhaltene wäßrige Lösung ohne weitere Behandlung in einem pH-Bereich, in dem die Kohlenhydrate und davon abgeleitete Carbonsäuren beständig sind, mit Sauerstoff als Oxidationsmittel in Gegenwart eines Katalysators, der wenigstens ein Platinmetall enthält, oxidiert.

Die vorliegende Erfindung betrifft Waschmittel enthaltend Äthercarbonsäuren, die durch Oxäthylierung von Kohlenhydraten und anschließende katalytische Oxidation hergestellt werden.

Wegen der eutrophierenden Wirkung in Gewässern ist die Verwendung von Phosphaten in Wasch- und Reinigungsmitteln in einer Reihe von Ländern gesetzlich beschränkt, z. T. sogar verboten. Daher ist inzwischen eine Vielzahl von Ersatzstoffen für die Phosphate, insbesondere für das Natriumtripolyphosphat, als Gerüststoff ("Builder") entwickelt und vorgeschlagen worden. Jedoch werden die wünschenswerten waschtechnischen Eigenschaften des Natriumtripolyphosphates bisher von keinem Einzelstoff im ganzen erreicht. Vielmehr sind lediglich Kombinationen von Gerüststoffen in der Lage, in erster Näherung die Wirkung der Phosphate zu erreichen. Nur relativ wenige Ersatzstoffe für Phosphate- oder besser gesagt Teilersatzstoffe - sind hinsichtlich ihrer ökologischen Eigenschaften voll befriedigend. Wenn sie die Eutrophierung der Gewässer auch nicht fördern, so haben sie z. T. aber doch Eigenschaften, die für die Umwelt als bedenklich angesehen werden müssen, wie die Remobilisierung von Schwermetallen aus den Sedimenten der Gewässer oder mangelnde biologische Abbaubarkeit; daher ist ihre Umweltrelevanz, auch wenn diese Stoffe nach heutigem Kenntnisstand zunächst einmal nicht als toxisch einzustufen sind, ungewiß. Die Suche nach effektiven Gerüststoffen für Waschmittel, die bezüglich ihrer ökologischen Wirkung als unbedenklich eingestuft werden können, geht daher weiter.

Aus der japanischen Offenlegungsschrift 58-117284 sind sogenannte Viskositätsverminderer von Steinkohlenschlämmen bekannt, die durch Umsetzung mehrwertiger Alkohole, zu denen auch Kohlenhydrate gerechnet werden, mit Alkylenoxiden Zu Polyätherverbindungen sowie gegebenenfalls anschließende Umwandlung der endständigen Hydroxygruppen Zu Carboxylgruppen hergestellt werden können; genauere Angaben über die Synthese dieser Produkte werden jedoch nicht gemacht.

Die Oxäthylierung von Kohlenhydraten ist seit längerem bekannt. So hat z.B. W. Gerhardt, J. f. prakt. Chem., 4. Reihe, 29, 300 (1965), die Oxäthylierung von Saccharose beschrieben; in der japanischen Offenlegungsschrift 58-117284 sind noch weitere Kohlenhydrate erwähnt. Jedoch sind wirtschaftliche Verfahren zur Herstellung oxidierter Oxäthylate von Kohlenhydraten ebenso wie die Verwendung solcher Produkte als Gerüststoff bisher nicht bekannt.

Es bestand daher die Aufgabe, ein Waschmittel zu entwickeln, das biologisch abbaubare Ersatzstoffe für Phosphate enthält.

Überraschenderweise wurde nun gefunden, daß man abbaubare Ersatzstoffe für Phosphate mit gutem Kalkbindevermögen erhält, wenn man Kohlenhydrate oxäthyliert, gegebenenfalls unter zusätzlicher Oxalkylierung durch ein höheres Alkylenoxyd, und die endständigen primären Hydroxylgruppen durch katalytische Oxidation in Carboxylgruppen überführt.

Gegenstand der Erfindung ist somit ein Waschmittel enthaltend Äthercarbonsäuren, die durch Oxalkylierung von Kohlenhydraten und deren Derivaten und anschließende Oxidation hergestellt werden, indem man die Kohlenhydrate oder deren Derivate in üblicher Weise entweder unmittelbar äthoxyliert oder zunächst mit einem höheren Alkylenoxid oxalkyliert und danach äthoxyliert und die dabei erhaltene wäßrige Lösung ohne weitere Behandlung in einem pH-Bereich, in dem die Kohlenhydrate und davon abgeleitete Carbonsäuren beständig sind, mit Sauerstoff als Oxidationsmittel in Gegenwart eines Katalysators, der wenigstens ein Platinmetall enthält, oxidiert.

Als Ausgangsmaterial für die Äthercarbonsäuren sind praktisch alle Kohlenhydrate mit mindestens einer oxalkylierbaren Hydroxygruppe und einer zu einer Carboxylgruppe oxidierbaren Alkoholgruppe geeignet, wie Glucose, Galactose, Maltose, Lactose, Fruktose, $\beta$-Cyclodextrin, insbesondere Saccharose. Als Derivate seien beispielsweise genannt $\alpha$-Methyl-D-glucosid, Sorbit, Mannit, 2-Desoxy-D-ribose und D-Glucosamin.

Die Oxäthylierung und Oxalkylierung erfolgen in üblicher Weise, und zwar im allgemeinen in wäßriger Lösung unter Einwirkung basischer Katalysatoren bei Temperaturen zwischen 50 und 150°C, vorzugsweise in einem Druckbereich von Atmosphärendruck bis 15 bar. Die Lösung, die die Verbindungen mit den endständigen Hydroxyäthylgruppen enthält, wird, vorzugsweise nach Verdünnung auf einen Wassergehalt von 70 bis 90 Gew.-% der katalytischen Oxidation unterworfen, wobei die Äthercarbonsäuren entstehen. Von den Hydroxylgruppen der Kohlenhydrate und der Derivate sollen zweckmäßig mindestens 50 alkoxyliert sein, vorzugsweise mindestens 75 und insbesondere etwa 100 %.

Als höhere Alkylenoxide kommen Butylenoxid, Styroloxid und vor allem Propylenoxid in Frage. Diese können z.B. in einer Menge von bis zu 3 Mol, zweckmäßig von mindestens 0,1 Mol verwendet werden. Naturgemäß ist der Anteil der höheren Alkylenoxide zweckmäßig so zu wählen, daß die biologische Abbaubarkeit gewährleistet bleibt.

Das Äthylenoxid wird zweckmäßig in einer Menge von mindestens 1 Mol, z.B. bis zu 10 Mol und vorzugsweise bis zu 5 Mol verwendet. Dabei beziehen sich alle Molangaben auf Gramm-Mol Hydroxylgruppen, die in dem Kohlenhydrat bzw. dessen Derivat enthalten sind. Sofern diese zunächst mit einem anderen Alkylenoxid als Äthylenoxid umgesetzt werden, beträgt die Gesamtmenge des umgesetzten Alkylenoxids, also einschließlich des Äthylenoxids, zweckmäßig nicht mehr als 5 Mol.

Als Katalysatoren eignen sich solche, die Platinmetalle, also Osmium, Iridium, Rhodium, Ruthenium, Palladium und/oder Platin, enthalten. Bevorzugt sind Katalysatoren, die eine Kombination von Palladium und Platin und insbesondere nur Platin enthalten. Vorzugsweise sind die Platinmetalle auf einem Träger, wie $Al_2O_3$ oder $SiO_2$, insbesondere auf Aktivkohle aufgebracht. Der Metallgehalt des Katalysators liegt im allgemeinen bei 1 bis 15, vorzugsweise bei 5 bis 10 Gew.-%.

Zuweilen kann es zweckmäßig sein, insbesondere wenn man Kohlenhydratderivate mit schlechterer Wasserlöslichkeit als Ausgangsstoffe verwendet, einen unter den Reaktionsbedingungen inerten Lösungsvermittler, vorzugsweise in einer Konzentration von 10 bis 75 Gew.-%, insbesondere 30 bis 50 Gew.-%, bezogen auf die Menge an Wasser und Lösungsvermittler, zuzusetzen. Geeignet sind vor allem solche Lösungsvermittler, die beim Durchleiten von Sauerstoff durch die wäßrige Lösung wenig flüchtig sind, so daß eine Explosionsgefahr im Dampfraum weitgehend vermieden wird; auf der anderen Seite sollte der Lösungsvermittler nach der Oxydation leicht abtrennbar sein, beispielsweise durch Destillation. Geeignete Lösungsvermittler sind beispielsweise Glykoläther ohne freie OH-Gruppen, wie solche der Formel $R^1O$-$(CHRCH_2O)_nR_2$, wobei n eine Zahl von 1 bis 4, R H oder $CH_3$ und $R^1$ und $R^2$ jeweils unabhängig voneinander $C_1$-$C_4$-Alkyl bedeuten. Besonders geeignet sind die Dimethyl-, Diäthyl- und Methyl-äthyl-äther der genannten allgemeinen Formel mit Siedepunkten im Bereich von 100 bis etwa 250°C, beispielsweise Triäthylenglykoldimethyläther und bevorzugt Diäthylenglykoldimethyläther.

Bevorzugtes Oxidationsmittel ist reiner Sauerstoff. Es können jedoch auch Mischungen von Sauerstoff mit unter den Reaktionsbedingungen inerten Gasen, beispielsweise Mischungen von Sauerstoff mit Inertgasen, verwendet werden. Natürlich ist auch Luft selbst geeignet.

In der Regel arbeitet man bei der Oxidation bei einem Gesamtdruck von 0,5 bis 100 bar. Die Reaktionsgeschwindigkeit steigt mit steigendem Sauerstoffpartialdruck deutlich an; jedoch kann der Vorteil der höheren Reaktionsgeschwindigkeit durch den bei Anwendung von höherem Druck erforderlichen höheren apparativen Aufwand in Bezug auf die Wirtschaftlichkeit überkompensiert werden. Bevorzugt ist ein Druckbereich von Atmosphärendruck bis 10 bar (absolut), wobei das Arbeiten bei Atmosphärendruck besonders einfach auszuführen ist.

Die Oxidation wird in der Regel bei einer Temperatur von 5 bis 800°C, vorzugsweise von 10 bis 60°C, insbesondere von 20 bis 40°C durchgeführt. Da viele Kohlenhydrate und davon abgeleitete Carbonsäuren im sauren Bereich wenig beständig sind, wird die Oxidation zweckmäßig im etwa neutralen bis schwach alkalischen Medium, also im pH-Bereich von 5 bis 9, vorzugsweise von 6 bis 8,5 und insbesondere von 7 bis 8, durchgeführt. Die während der Oxydation entstehenden Carbonsäuren werden zweckmäßig abgefangen, z.B. durch geeignete Puffersubstanzen oder vorteilhaft durch Zugabe von wäßrigen Basen, etwa Alkali oder Erdalkalihydroxyd-Lösungen, wobei diese zweckmäßig so zudosiert werden, daß der pH-Wert des Reaktionssystems während der Oxydation im Bereich von 6 bis 9 bleibt. Bei einer vollständigen Neutralisation fallen die Oxydationsprodukte dann als Salze an.

Das Verfahren zur Herstellung der Äthercarbonsäuren kann in allen Apparaturen, die sich für die Durchführung von Reaktionen in der flüssigen Phase mit oder ohne Anwendung von Überdruck eignen, durchgeführt werden. Beispiele dafür sind die Durchführung in einem Rührkessel oder in einer Blasensäule mit suspendiertem Katalysator. Die Oxydation kann aber auch an einem Festbett mit gekörntem Katalysator in einem Rieselphasenreaktor durchgeführt werden.

Die erforderliche Reaktionszeit wird zweckmäßig dadurch ermittelt, daß man in gewissen Zeitabständen Proben der Reaktionslösung entnimmt und analysiert. Beispielsweise kann die Ausbeute der Reaktionsprodukte auf einfache Weise durch Analyse einer Probe mit Hilfe der Hochdruckflüssigkeitschromatographie im Vergleich zu Standardlösungen laufend bestimmt werden. Die Optimierung der Reaktionszeit ist zu empfehlen, da eine unnötig verlängerte Einleitung von Sauerstoff zu Überoxydationen und damit beispielsweise zu Decarboxylierungen und zur Verminderung der Ausbeute an den gewünschten Reaktionsprodukten führen kann.

Das Reaktionsgemisch kann nach üblichen Methoden aufgearbeitet werden. Z.B. werden zunächst das Wasser und etwa vorhandene Lösungsvermittler destillativ entfernt. Anschließend wird eine Reinigung, z.B. durch Chromatographie, Kristallisation oder Fällung, vorgenommen. Auch eine Abtrennung aus der bei der Oxydation erhaltenen Lösung über basische Ionenaustauscher in der OH-Form ist möglich. Im allgemeinen hat es sich jedoch als vorteilhaft erwiesen, die bei der Reaktion erhaltene Lösung einer Sprühtrocknung zu unterwerfen.

Das Kalkbindevermögen der Äthercarbonsäuren kann durch übliche Analysenmethoden ermittelt werden, beispielsweise durch Trübungstitration bzw. potentiometrische Titration mittels einer ionenspezifischen Elektrode.

Die nachfolgenden Beispiele erläutern die Erfindung.

Beispiel 1 - Herstellung

In einem 2 l-Rührautoklaven wurden 111 g Saccharose und 2,75 g KOH in 450 ml Wasser mit 333 g Äthylenoxid so versetzt, daß die Temperatur zwischen 85 und 100°C lag und der Druck bis zu 4 bar betrug. Nach 2 Stunden Reaktionszeit wurde die Lösung auf Raumtemperatur abgekühlt und mit Wasser auf 4 l Gesamtvolumen aufgefüllt. Diese Lösung wurde in einem von außen beheizten senkrecht angeordneten Glasrohr (Durchmesser 100 mm, Länge 800 mm) nach Zugabe von 20 g eines handelsüblichen Katalysators (5 Platin auf Aktivkohle) bei 500°C von unten durch eine Glasfritte mit 100 NL/h Sauerstoff begast. Durch kontinuierliche Zugabe von 30 %iger wäßriger

Natronlauge wurde der pH-Wert bei 7 bis 7,5 gehalten. Die Oxidationszeit betrug 6 Stunden. Durch Sprühtrocknung des Reaktionsproduktes erhielt man 390 g eines farblosen Pulvers mit einem Kalkbindevermögen von 279 mg $CaCO_3$ pro g Substanz (ermittelt bei Raumtemperatur).

Beispiel 2 - Herstellung

Analog Beispiel 1 wurden 110 g Sorbit mit 470 g Ethylenoxid und 4 g KOH in 950 ml Wasser ethoxyliert und anschließend nach Zugabe von 4,27 l Wasser und 290 g Katalysator oxidiert. Das sprühgetrocknete Produkt (484 g) hatte ein Kalkbindevermögen von 373 mg $CaCO_3$/g Substanz.

Beispiel 3 - Herstellung

Analog Beispiel 1 wurden Pentaerythrit und $\alpha$-Methylglucosid ethoxyliert und oxidiert und nach Aufarbeitung hinsichtlich ihres Kalkbindevermögens untersucht. Das mit Pentaerythrit erhaltene Produkt besaß ein Kalkbindevermögen von 343 mg $CaCO_3$/g Substanz und das mit $\alpha$-Methylglucosid ein solches von 327 mg $CaCO_3$/g Substanz.

Beispiel 4 - Verwendung

Die Überlegenheit des erfindungsgemäß erhaltenen Produkts als Gerüstbildner zeigt sich in den Prüfergebnissen von Waschmittelformulierungen, in denen lediglich die nach der DE-OS 35 35 720 erhaltene Saccharose-tricarbonsäure durch das erfindungsgemäße Produkt gemäß Beispiel 1 substituiert wurde. Die waschtechnische Prüfung dieser Waschmittel erfolgte nach den anerkannten Regeln der Technik in Anlehnung an DIN 44983:
Die Waschkraft (Remissionsdifferenz) wurde durch Remissionsmessung (Farbmeßgerät RFG 3 der Fa. Zeiss) an zwei WFK- und EMPA-Test-Schmutzgeweben bei einer Wellenlänge von 460 nm photometrisch ermittelt (WFK = Wäscherei-Forschungsinstitut Krefeld; EMPA = Eidgenössische Material-Prüfanstalt, Schweiz). Hierbei wurde die "Differenzmethode" entsprechend der Gleichung:

$$\% \ R \ = \ \% \ R_g \ - \ \% \ R_u \ \text{angewendet.}$$

Darin ist
% R = Remissionsdifferenz (Waschkraft),
% Rg = Remission des gewaschenen Gewebes
% Ru = Remission des ungewaschenen Gewebes
Die Gewebeablagerung (Inkrustation) wurde in Form der anorganischen Gewebeasche als prozentualer Glührückstand bei 800°C bestimmt.
Die Herstellung der Waschmittelpulver wurde teils nach dem sogenannten Heißsprühverfahren und teils nach dem sogenannten Sprühnebelmischverfahren (Trockenmischprozeß) durchgeführt. Die Heißsprühung erfolgte mittels eines Laborsprühtrockners (Fa. Büchi, Typ 190) mit einer Eingangstemperatur von ca. 180°C, einer Ausgangstemperatur von ca. 100°C, einem Sprühdruck von 5 bar und einer Feststoffkonzentration von 30 Gew.-%.
Bei dem Sprühnebelmischverfahren kam ein Freifallmischer zur Anwendung, wobei die flüssigen Bestandteile mittels einer geeigneten Sprühvorrichtung auf die pulverförmigen Trockenbestandteile aufgedüst wurden. Die Arbeitsweise ist im einzelnen in "Seifen, Fette, Öle, Wachse", 99, (1973), 351 bis 357, beschrieben.
Waschmittelformulierungen und Waschversuche sind in den folgenden Tabellen beschrieben.

| Waschmittelformulierungen | | |
|---|---|---|
| | Sprühnebelmischversuche | |
| | Beispiel | |
| | Vergleich | 4 |
| Saccharose-tricarbonsäure (STA) (bekanntes Produkt wie in DE-OS 35 35 720 beschrieben) | 10,0 | - |
| Erfindungsgemäße Äthercarbonsäure (wie Beispiel 1) | - | 10,0 |
| Zeolith | 21,5 | 21,5 |
| Na-Perborat-Tetrahydrat | 20,0 | 20,0 |
| Anionische Tenside (Alkylbenzolsulfonate) | 7,0 | 7,0 |
| Nichtionische Tenside (oxalkylierte Alkohole) | 4,0 | 4,0 |
| Seife | 3,5 | 3,5 |
| Na-Silicat | 5,0 | 5,0 |
| Carboxymethylcellulose | 1,0 | 1,0 |
| Methylcellulose Rest übliche Waschmittel-bestandteile auf 100 % | 0,5 | 0,5 |
| | Rest übliche Waschmittel-bestandteile auf 100 % | |

**Waschversuche nach DIN 44983 (Zweilaugenverfahren, 60°C-Wäsche, Wasserhärte 18°d)**

| Beispiel | Dosierung (g) Vor-/Klarwäsche | Primärwaschwirkung an verschiedenen Testgeweben (% Remissionsdifferenz) | | |
| --- | --- | --- | --- | --- |
| | | EMPA Baumwolle Nr. 101 | WFK Baumwolle Nr. 10 C | WFK Baumwolle Nr. 10 D |
| Vergleich | 150/150 | 26 | 21 | 23 |
| 4 | 150/150 | 31 | 27 | 30 |

**Waschversuche nach DIN 44983 (Zweilaugenverfahren, 90°C-Wäsche, Wasserhärte 18°d)**

| Beispiel | Dosierung (g) Vor-/Klarwäsche | % Asche nach 25 Waschzyklen | | |
| --- | --- | --- | --- | --- |
| | | Frottee | Baumwolle (EMPA) | Doppelripp (WFK) |
| Vergleich | 150/150 | 2,1 | 2,3 | 2,4 |
| 4 | 150/150 | 0,9 | 0,4 | 0,4 |

## Patentansprüche

1. Waschmittel enthaltend Äthercarbonsäuren, die durch Oxalkylierung von Kohlenhydraten und deren Derivaten und anschließende Oxidation hergestellt werden, indem man die Kohlenhydrate oder deren Derivate in üblicher Weise entweder unmittelbar äthoxyliert oder zunächst mit einem höheren Alkylenoxid alkoxyliert und dann äthoxyliert und die dabei erhaltene wäßrlge Lösung ohne weitere Behandlung in einem pH-Bereich, in dem die Kohlenhydrate und davon abgeleitete Carbonsäuren beständig sind, mit Sauerstoff als Oxidationsmittel in Gegenwart eines Katalysators, der wenigstens ein Platinmetall enthält, oxidiert.

6

2. Waschmittel nach Anspruch 1, dadurch gekennzeichnet, daß bei der Herstellung der Äthercarbonsäuren die Menge des höheren Alkylenoxids bis zu 3 Mol und die des Äthylenoxids von 1 bis 10 Mol, jeweils bezogen auf 1 Gramm-Mol OH in dem Kohlenhydrat und dem Derivat davon, beträgt, wobei die Gesamtmenge an Alkylenoxiden vorzugsweise bis zu 5 Mol beträgt.

3. Waschmittel nach Anspruch 1 oder 2, dadurch gekennzeichnet, daß bei der Herstellung der Äthercarbonsäuren der Katalysator als Platinmetall eine Kombination von Palladium und Platin, insbesondere nur Platin enthält.

4. Waschmittel nach einem oder mehreren der Ansprüche 1 bis 3, dadurch gekennzeichnet, daß bei der Herstellung der Äthercarbonsäuren der Katalysator als 1 bis 15 Gew.-%, vorzugsweise 5 bis 10 Gew.-%, des Platinmetalls und einem Träger, vorzugsweise Aktivkohle, besteht.

5. Waschmittel nach einem oder mehreren der Ansprüche 1 bis 4, dadurch gekennzeichnet, daß bei der Herstellung der Äthercarbonsäuren die Oxydation in einem Druckbereich von 0,5 bis 100 bar, vorzugsweise von Atmosphärendruck bis 10 bar, insbesondere bei Atmosphärendruck, durchgeführt wird.

6. Waschmittel nach einem oder mehreren der Ansprüche 1 bis 5, dadurch gekennzeichnet, daß bei der Herstellung der Äthercarbonsäuren die wäßrige Lösung einen unter den Reaktionsbedingungen inerten Lösungsvermittler, vorzugsweise in einer Menge von 10 bis 75 Gew.-%, insbesondere 30 bis 50 Gew.-%, enthält, wobei der Lösungsvermittler vorzugsweise ein Glykoläther ohne Hydroxygruppen, insbesondere Diäthylenglykoldimethyläther ist.

7. Waschmittel nach einem oder mehreren der Ansprüche 1 bis 6, dadurch gekennzeichnet, daß bei der Herstellung der Äthercarbonsäuren die Oxydation bei einer Temperatur von 5 bis 80°C, vorzugsweise von 10 bis 60°C, insbesondere von 20 bis 40°C, durchgeführt wird.

8. Waschmittel nach einem oder mehreren der Ansprüche 1 bis 7, dadurch gekennzeichnet, daß bei der Herstellung der Äthercarbonsäuren die Oxydation bei einem pH-Wert von 5 bis 9, vorzugsweise von 6 bis 8,5 und insbesondere von 7 bis 8 durchgeführt wird.

9. Verfahren zur Herstellung eines Waschmittels nach einem oder mehreren der Ansprüche 1 bis 8, dadurch gekennzeichnet, daß die Komponenten nach dem Heißsprühverfahren vermischt werden.

10. Verfahren zur Herstellung eines Waschmittels nach einem der Ansprüche 1 bis 8, dadurch gekennzeichnet, daß die Komponenten nach dem Sprühnebelmischverfahren vermischt werden.